# EUROPEAN PATENT APPLICATION

(11) **EP 4 751 727 A1**
(43) Date of publication of application: **03.06.2026**
(21) Application number: 25217952.8
(22) Date of filing: 24.11.2025
(51) Int. Cl.: A61K 36/284, A61K 36/355, A61K 36/484, A61K 36/489, A61K 36/75, A61K 36/756, A61K 36/80, A61K 36/8994, A61K 36/21, A61K 35/64, A61P 17/00, A61K 36/36

(54) **TRADITIONAL CHINESE MEDICINE COMPOSITION FOR TREATING PEDIATRIC ATOPIC DERMATITIS AND METHOD FOR PREPARING THE SAME**

(30) Priority: 27.11.2024 CN 202411716716
(71) Applicant: Jiuhua Huayuan Pharmaceutical Co., Ltd., Anhui 239000 (CN)
(72) Inventor: JIA, Liujin, Chuzhou, Anhui (CN); GUAN, Yueqin, Chuzhou, Anhui (CN); CHEN, Lingwu, Chuzhou, Anhui (CN); JU, Zhihe, Chuzhou, Anhui (CN); LIU, Yadong, Chuzhou, Anhui (CN); LUO, Xuefeng, Chuzhou, Anhui (CN); ZHANG, Gusi, Chuzhou, Anhui (CN)
(74) Representative: Viering, Jentschura & Partner mbB Patent- und Rechtsanwälte

(57) **Abstract**

The present application discloses a traditional Chinese medicine composition for treating pediatric atopic dermatitis and a method for preparing the same, relating to the technical field of traditional Chinese medicine compositions. The traditional Chinese medicine composition incudes the following components of traditional Chinese medicinal raw materials by mass: 4 to 8 parts of an Atractylodes Rhizoma, 6 to 10 parts of a Lonicerae Japonicae Flos, 4 to 8 parts of a Phellodendri Chinensis Cortex, 4 to 8 parts of a Coicis Semen, 4 to 8 parts of an Achyranthis Bidentatae Radix, 2 to 8 parts of a Sophorae Flaves Centis Radix, 4 to 8 parts of a Dictamni Cortex, 4 to 8 parts of a Kochiae Fructus, 2 to 8 parts of a Cicadae Periostracum, and 2 to 8 parts of a Glycyrrhizae Radix et Rhizoma. The preparation method includes the following steps: immersing the traditional Chinese medicinal raw materials in water, heating under reflux for extraction, and filtering a resulting mixture to collect a filtrate; and concentrating the filtrate into a clear paste, cooling the clear paste, adding ethanol, letting a resulting mixture stand, collecting a supernatant, recovering ethanol, and performing a second concentrating on a resulting liquid to obtain a traditional Chinese medicine paste; and granulating the traditional Chinese medicine paste to obtain a product of the traditional Chinese medicine composition for treating pediatric atopic dermatitis. Through a combination of traditional Chinese medicine theory and modern pharmacology, the composition of the present application acts to clear heat and remove dampness, strengthen the spleen and promote the resolution of dampness, as well as dispel wind and relieve itching. In clinical practice, the composition of the present application has demonstrated significant efficacy in improving and managing symptoms of pediatric atopic dermatitis, characterized by a rapid onset of action, a short treatment course, low incidence of side effects, and no recurrence post-recovery.

## Description

### TECHNICAL FIELD

The present application relates to the technical field of traditional Chinese medicine composition, and more specifically to a traditional Chinese medicine composition for treating pediatric atopic dermatitis and a method for preparing the same.

### BACKGROUND

Pediatric atopic dermatitis (AD), also known as "atopic eczema," "hereditary allergic dermatitis," "atopic eczema," or "atopic eczema-dermatitis syndrome (AEDS)", is a chronic, relapsing, inflammatory skin disease associated with a genetic predisposition to allergies. It is clinically characterized by pruritus, polymorphic skin lesions with exudative tendencies, and is frequently associated with asthma and allergic rhino-conjunctivitis. A key characteristic is the presence of a distinct "atopic" trait in the individual or their family. The global incidence rate is approximately 10%-20% in children and 1%-3% in adults, with a trend of increasing incidence. Due to its recurrent nature and resistance to cure, atopic dermatitis significantly impacts patients' physical and mental health and quality of life.

The pathogenesis of pediatric atopic dermatitis is complex. From a Western medical perspective, it is primarily attributed to an imbalance in the Th1/Th2 cell ratio. Previous studies on the acute phase of AD have found that patients exhibit a dual expression pattern of Th1/Th2 cytokines in their peripheral blood. Overexpression of IL-4, one of the key cytokines in the Th2 pathway, is responsible for the typical clinical manifestations of AD. IL-31, on the other hand, has been demonstrated to induce the expression of multiple eczema-related chemokines, thus provoking skin inflammation and serving as a key mediator of pruritus (itching). Consequently, conventional modern medical treatments for AD primarily include antihistamines, corticosteroids, antimicrobial agents, and immunosuppressants. However, the associated side effects and adverse reactions often lead to poor long-term medication adherence, resulting in suboptimal long-term efficacy.

Traditional Chinese medicine (TCM) has a long history, remarkable efficacy, and a low incidence of adverse reactions. In recent years, with growing worldwide interest in TCM, advances have been made in its application for treating AD. Numerous publications and patents have disclosed relevant TCM formulations for pediatric atopic dermatitis. However, many existing formulations still face challenges, including inadequate efficacy, high recurrence rates, and extended treatment durations. Therefore, developing a TCM preparation that can overcome these limitations is of great importance.

### SUMMARY

The purpose of the present application is to provide a traditional Chinese medicine composition for treating pediatric atopic dermatitis and a method for preparing the same. The composition when used in the treatment for pediatric atopic dermatitis has the advantages of rapid efficacy, minimal side effects, and no recurrence upon drug withdrawal. Moreover, it is prepared from low-cost raw materials using a simple process.

To solve the above-mentioned technical problems, the following technical solutions are adopted:
In a first aspect of the present application, a traditional Chinese medicine composition for treating pediatric atopic dermatitis is provided. The traditional Chinese medicine composition for treating pediatric atopic dermatitis comprises the following components of traditional Chinese medicinal raw materials by mass: 4 to 8 parts of an Atractylodes Rhizoma, 6 to 10 parts of a Lonicerae Japonicae Flos, 4 to 8 parts of a Phellodendri Chinensis Cortex, 4 to 8 parts of a Coicis Semen, 4 to 8 parts of an Achyranthis Bidentatae Radix, 2 to 8 parts of a Sophorae Flaves Centis Radix, 4 to 8 parts of a Dictamni Cortex, 4 to 8 parts of a Kochiae Fructus, 2 to 8 parts of a Cicadae Periostracum, and 2 to 8 parts of a Glycyrrhizae Radix et Rhizoma.

In another aspect of the present application, a method for preparing the traditional Chinese medicine composition for treating pediatric atopic dermatitis is provided. The method comprises the following steps:
step S1, immersing the traditional Chinese medicinal raw materials in water, heating under reflux for extraction, and filtering a resulting mixture to collect a filtrate; and
step S2, concentrating the filtrate into a clear paste, cooling the clear paste, adding ethanol, letting a resulting mixture stand, collecting a supernatant, recovering ethanol, and performing a second concentrating on a resulting liquid to obtain a traditional Chinese medicine paste; and granulating the traditional Chinese medicine paste to obtain a product of the traditional Chinese medicine composition for treating pediatric atopic dermatitis.

Atractylodes Rhizoma: This medicinal material is irregularly moniliform or nodular-cylindrical in shape, slightly curved, and occasionally branched, measuring 3-10 cm in length and 1-2 cm in diameter. The surface is grayish-brown, with wrinkles, transverse furrows, and remnants of fibrous roots. The apex exhibits stem scars or residual stem bases. It is firm in texture. The fractured surface is yellowish-white or grayish-white, scattered with numerous orange-yellow or brownish-red oil cavities. Upon prolonged exposure, fine white needle-like crystals may effloresce. It has a distinct aromatic odor and a taste that is slightly sweet, pungent, and bitter.

Lonicerae Japonicae Flos: This medicinal material is clavate, thicker at the upper end and thinner toward the base, and slightly curved, measuring 2-3 cm in length, approximately 3 mm in diameter at the top and about 1.5 mm at the bottom. The surface is yellowish-white or greenish-white (the color deepens with prolonged storage) and is densely pubescent. Leaf-like bracts are occasionally present. The calyx is green and 5-lobed at the apex; the lobes are hairy and about 2 mm long. In opened flowers, the corolla is tubular with a bilabiate apex. There are five stamens adnate to the corolla tube, yellow in color, and a single pistil with a glabrous ovary. It has a delicate, fragrant odor and a bland, slightly bitter taste.

Phellodendri Chinensis Cortex: This medicinal material occurs as plate-like or shallowly grooved pieces, varying in length and width, and 1-6 mm thick. The outer surface is yellowish-brown or yellow-brown, flat or with longitudinal grooves, and occasionally shows lenticel scars and remnants of grayish-brown coarse bark. The inner surface is dark yellow or light brown, with fine, dense longitudinal ridges. It is light in weight and hard in texture. The fractured surface is fibrous, exhibits a laminated (splintery) structure, and is deep yellow in color. It has a faint odor and an extremely bitter taste. When chewed, it feels sticky due to the presence of mucilage.

Coicis Semen: This medicinal material is broadly ovate or oblong, measuring 4-8 mm in length and 3-6 mm in width. The surface is milky white and smooth, occasionally with remnants of a yellowish-brown seed coat. One end is blunt and rounded, while the other is broader and slightly concave, with a light brown, dot-like hilum. The dorsum is rounded and convex, and the venter bears a broad, deep longitudinal furrow. The texture is firm. The fractured surface is white and farinaceous (starchy). It has a faint odor and a slightly sweet taste.

Achyranthis Bidentatae Radix (from Huaiqing region): This medicinal material is a slender cylindrical shape, straight or slightly curved, 15-70 cm long and 0.4-1 cm in diameter. The surface is grayish-yellow or light brown, with slightly twisted fine longitudinal wrinkles, sparsely arranged lateral root scars, and transverse lenticel-like protrusions. It is hard and brittle, easily broken, softening when damp. The fracture surface is flat, light brown, slightly horny and oily, with a relatively large, yellowish-white xylem in the central vascular bundle, surrounded by numerous yellowish-white dot-like vascular bundles arranged discontinuously in 2-4 rings. It has a faint odor and a slightly sweet and slightly bitter taste.

Sophorae Flaves Centis Radix: This medicinal material is long cylindrical, frequently with branches in the lower part, measuring 10-30 cm in length and 1-6.5 cm in diameter. The surface is grayish-brown or brownish-yellow, with longitudinal wrinkles and transverse lenticel-like protrusions. The outer bark (cork layer) is thin, frequently cracked and recurved, and exfoliates easily, exposing a smooth, yellow surface underneath. It is hard in texture and not easily broken. The fracture is fibrous. Slices are 3-6 mm thick, with a yellowish-white cut surface showing radial texture (striations) and fissures; some pieces show anomalous vascular bundles arranged in concentric rings or scattered irregularly. It has a faint odor and an extremely bitter taste.

Dictamni Cortex: This medicinal material occurs in quilled (rolled-tube) forms, measuring 5-15 cm in length, 1-2 cm in diameter, and 0.2-0.5 cm in thickness. The outer surface is grayish-white or pale grayish-yellow, with fine longitudinal wrinkles and fine root scars, and often exhibits raised granular dots. The inner surface is off-white, with fine longitudinal striations. It is brittle and breaks with a puff of dust. The fracture is uneven and somewhat laminated. When the outer layer is peeled away, small, sparkling bright spots become visible against the light. It has a distinctive goat-like (caprylic) odor and a slightly bitter taste.

Kochiae Fructus: This medicinal material is a flattened spherical, five-pointed star-shaped fruit, 1-3 mm in diameter. It is enveloped by a persistent perianth and is grayish-green or light brown in color, with five membranous wings around the periphery. On the dorsal side, the center exhibits a slightly raised, dot-like fruit stalk scar, from which 5-10 radial veins extend. Peeling off the perianth reveals a membranous, translucent pericarp. The seeds are flattened-ovoid, about 1 mm long, and black. It has a faint odor and a slightly bitter taste.

Cicadae Periostracum: This medicinal material is slightly elliptical and curved, measuring approximately 3.5 cm in length and 2 cm in width. The surface is yellowish-brown, translucent, and glossy. The head bears a pair of filamentous antennae, mostly broken off, and prominent compound eyes. The frons has a protruding apex. The mouthparts are well-developed, featuring a broad, short labrum and an elongated, tubular labium. The dorsal side of the thorax has a cross-shaped fissure, the margins of which are incurred; two pairs of small wings are present on either side of the dorsal ridge. The ventral surface has three pairs of legs covered with fine yellowish-brown hairs. The abdomen is bluntly rounded and consists of nine segments. It is light in weight, hollow, and fragile. It has a faint odor and a bland taste.

Glycyrrhizae Radix et Rhizoma: The root of this medicinal material is cylindrical, 25-100 cm long and 0.6-3.5 cm in diameter. The outer bark (cork) varies in texture from tight to loose. The surface is reddish-brown or grayish-brown, with prominent longitudinal wrinkles, grooves, lenticels, and sparse fine root scars. The texture is firm. The fracture is slightly fibrous, yellowish-white, and farinaceous (starchy), showing a distinct cambium ring, radial rays, and occasionally fissures. The rhizome is cylindrical, with bud scars on the surface and pith in the center of the cross-section. It has a faint odor and a sweet and distinctive taste.

This application posits that the etiology and pathology of pediatric atopic dermatitis lie in a child's constitutional hypersensitivity, coupled with impaired transportive function of the spleen and stomach. This leads to an internal accumulation of fetal fire damp-heat, which, when combined with external invasion of wind-damp-heat pathogens, accumulates and stagnates in the skin, thereby causing the disease. The exudative type often occurs in overweight children and is characterized by dampness predominating over heat; the dry type is commonly seen in thin or older children and is characterized by heat predominating over dampness. In the acute stage, the condition is characterized by fetal fire damp-heat. In the subacute stage, spleen deficiency fails in its transport function, leading to internal accumulation of damp-heat. In the chronic stage, there is a failure of qi and blood to moisten the tissues, allowing wind pathogens to linger. Overall, damp-heat is considered the primary pathogenic factor. The treatment principles are to: clear Heat and remove dampness, fortify the spleen and percolate dampness, and dispel wind to relieve itching.

Therefore, the medication principle of the traditional Chinese medicine composition for treating pediatric atopic dermatitis provided by the present application is as follows: in this formula, Atractylodes Rhizoma, Phellodendri Chinensis Cortex, and Lonicerae Japonicae Flos serve as the sovereign herbs. Atractylodes Rhizoma, being bitter and warm, effectively strengthens the spleen and dries dampness, while also counteracting the cold nature of other ingredients. Phellodendri Chinensis Cortex, bitter and cold, uses its bitterness to dry dampness and its coldness to clear heat. Lonicerae Japonicae Flos, sweet and cold, is a commonly used heat-clearing and detoxifying herb in external medicine. The minister herbs are Coicis Semen, Achyranthis Bidentatae Radix from Huaiqing region, and Sophorae Flavescentis Radix. Coicis Semen, sweet, bland, and slightly cold, promotes diuresis to leach out dampness and assists Atractylodes Rhizoma in strengthening the spleen. Achyranthis Bidentatae Radix from Huaiqing region, bitter, sour, and neutral, promotes urination, relieves strangury, and guides the damp-heat pathogen downward. Sophorae Flavescentis Radix, bitter and cold, clears heat and dries dampness, dispels wind and kills parasites. It is a key herb for treating various skin diseases, and modern pharmacological studies indicate it possesses anti-allergic effects. The assistant and courier herbs are Dictamni Cortex, Kochiae Fructus, Cicadae Periostracum, and Glycyrrhizae Radix et Rhizoma. Both Dictamni Cortex and Kochiae Fructus clear heat and remove dampness, as well as dispel wind to relieve itching. Cicadae Periostracum disperses wind-heat, dispels wind to relieve itching, and has a sedative function. Modern pharmacological studies have shown that it exhibits anti-allergic and immunosuppressive effects. Glycyrrhizae Radix et Rhizoma serves as the courier herb to harmonize all the other herbs.

Based on the above mechanism, the product of the traditional Chinese medicine composition for treating pediatric atopic dermatitis in the present application can also be used to treat purpura, infantile eczema, impetigo, oral ulcers, and adenoid hypertrophy in children.

Compared to the prior art, embodiments of the present application have at least the following advantages or beneficial effects:
Theoretically, the traditional Chinese medicine composition for treating pediatric atopic dermatitis of the present application employs Atractylodes Rhizoma, Phellodendri Chinensis Cortex, and Lonicerae Japonicae Flos as the sovereign herbs; Coicis Semen, Achyranthis Bidentatae Radix from Huaiqing region, and Sophorae Flavescentis Radix as the minister herbs; and Dictamni Cortex, Kochiae Fructus, Cicadae Periostracum, and Glycyrrhizae Radix et Rhizoma as the assistant and courier herbs. Through a combination of traditional Chinese medicine theory and modern pharmacology, the composition of the present application acts to clear heat and remove dampness, strengthen the spleen and promote the resolution of dampness, as well as dispel wind and relieve itching. In clinical practice, the composition of the present application has demonstrated significant efficacy in improving and managing symptoms of pediatric atopic dermatitis, characterized by a rapid onset of action, a short treatment course, low incidence of side effects, and no recurrence post-recovery, ultimately providing a favorable clinical outcome for these patients.

### DETAILED DESCRIPTION OF THE EMBODIMENTS

To clarify the objectives, technical solutions, and advantages of the embodiments of the present application, the following is a detailed description of the technical solutions in the embodiments. If specific conditions are not stated in the embodiments, conventional conditions or those recommended by manufacturers should be followed. Reagents or instruments not specified with manufacturers are standard products available commercially.

It is noted that, where there is no conflict, the embodiments and features within the embodiments of the present application can be combined with each other. The following provides detailed explanations of the present application with reference to specific embodiments.

A traditional Chinese medicine composition for treating pediatric atopic dermatitis comprises the following components of traditional Chinese medicinal raw materials by mass: 4 to 8 parts of an Atractylodes Rhizoma, 6 to 10 parts of a Lonicerae Japonicae Flos, 4 to 8 parts of a Phellodendri Chinensis Cortex, 4 to 8 parts of a Coicis Semen, 4 to 8 parts of an Achyranthis Bidentatae Radix, 2 to 8 parts of a Sophorae Flaves Centis Radix, 4 to 8 parts of a Dictamni Cortex, 4 to 8 parts of a Kochiae Fructus, 2 to 8 parts of a Cicadae Periostracum, and 2 to 8 parts of a Glycyrrhizae Radix et Rhizoma.

In some embodiments of the present application, the traditional Chinese medicine composition comprises the following components of traditional Chinese medicinal raw materials by mass: 6 parts of the Atractylodes Rhizoma, 8 parts of the Lonicerae Japonicae Flos, 6 parts of the Phellodendri Chinensis Cortex, 6 parts of the Coicis Semen, 6 parts of the Achyranthis Bidentatae Radix, 5 parts of the Sophorae Flaves Centis Radix, 6 parts of the Dictamni Cortex, 6 parts of the Kochiae Fructus, 5 parts of the Cicadae Periostracum, and 5 parts of the Glycyrrhizae Radix et Rhizoma.

In some embodiments of the present application, the Achyranthis Bidentatae Radix is Achyranthis Bidentatae Radix from Huaiqing region.

A method for preparing the traditional Chinese medicine composition for treating pediatric atopic dermatitis, comprises the following steps:
step S1, immersing the traditional Chinese medicinal raw materials in water, heating under reflux for extraction, and filtering a resulting mixture to collect a filtrate; and
step S2, concentrating the filtrate into a clear paste, cooling the clear paste, adding ethanol, letting a resulting mixture stand, collecting a supernatant, recovering ethanol, and performing a second concentrating on a resulting liquid to obtain a traditional Chinese medicine paste; and granulating the traditional Chinese medicine paste to obtain a product of the traditional Chinese medicine composition for treating pediatric atopic dermatitis.

In some embodiments of the present application, in step S1, a ratio of the traditional Chinese medicinal raw materials to water is 1:(6-14), an immersing temperature is room temperature, and the immersing time is 0.5 to 3 hrs; and the heating under reflux for extraction is performed twice, for 1 to 3 hrs each time, and filtrates obtained from the twice heating under reflux for extraction are combined and subjected to concentrating in step S2.

In some embodiments of the present application, in step S2, the concentrating is performed under a reduced pressure, a vacuum degree of the concentrating is -0.09 MPa to - 0.04 MPa, a temperature the concentrating is 60 to 80°C, and a clear paste obtained from the concentrating has a relative density of 1.10 to 1.25.

In some embodiments of the present application, the resulting mixture after adding ethanol in step S2 has an ethanol content of 30-80%, and a standing time is 8 hrs to 48 hrs.

In some embodiments of the present application, the second concentrating in step S2 is performed under a reduced pressure, the traditional Chinese medicine paste obtained from the second concentrating is a thick paste having a relative density of 1.25 to 1.36, an excipient is added to and mixed with the thick paste, granulated, followed by drying to form the product of the traditional Chinese medicine composition.

In some embodiments of the present application, the excipient comprises: a sweetener, a flavoring agent, and/or a bitterness inhibitor.

The sweetener comprises: glucose, fructose, sucrose, starch syrup, xylitol, monosaccharide syrup, fruit juice syrup, honey, stevia leaf extract, sorbitol, mannitol, D-tagatose, steviol glycoside, aspartame, saccharin, cyclamate, aspartame, acesulfame potassium, alitame, neotame, sucralose, isomaltitol, and novel saccharide (fructo-oligosaccharides), etc.

The flavoring agent comprises: peppermint oil, cinnamon oil, fennel oil, peppermint water, compound cardamom oleoresin, banana flavoring, pineapple flavoring, orange flavoring, lemon flavoring, chocolate flavoring, glucose flavoring, red apple flavoring, strawberry flavoring, and peach flavoring.

The bitterness inhibitor comprises: caffeic acid, ferulic acid, citric acid, adenosine monophosphate, phosphatidic acid, protein-phosphatidic acid complex, tannic acid, and neohesperidin.

In some embodiments of the present application, the second concentrating in step S2 is performed under a reduced pressure, the traditional Chinese medicine paste obtained from the second concentrating is a clear paste having a relative density of 1.05 to 1.25, which is granulated and dried to obtain a product of the traditional Chinese medicine composition product.

The features and properties of the present application are further described in detail below with reference to the following examples.

### Example 1

This example provides a traditional Chinese medicine composition for treating pediatric atopic dermatitis, which was prepared by the following steps:
In step S1, the following Chinese medicinal materials were collected as raw materials: 6 g of Atractylodes Rhizoma, 8 g of Lonicerae Japonicae Flos, 6 g of Phellodendri Chinensis Cortex, 6 g of Coicis Semen, 6 g of Achyranthis Bidentatae Radix, 5 g of Sophorae Flaves Centis Radix, 6 g of Dictamni Cortex, 6 g of Kochiae Fructus, 5 g of Cicadae Periostracum, 5 g of Glycyrrhizae Radix et Rhizoma; the above raw materials were added to 10 times an amount of water and immersed for 2 hrs, then heated under reflux for extraction twice, 2 hrs each time, and filtered, and the two filtrates were combined;
In step S2, the combined filtrate was concentrated under a reduced pressure at a temperature of 75°C and a vacuum degree of -0.06 MPa to obtain a clear paste having a relative density of 1.15. The clear paste was cooled, and ethanol was added to and mixed with the clear paste until an ethanol content reached 55%, a resulting mixture was allowed to stand for 24 hrs. A supernatant was collected, filtered, ethanol was recovered from a resulting filtrate, which was then subjected to concentrating under a reduced pressure at a temperature of 70°C and a vacuum degree of -0.06 MPa to yield a thick paste having a relative density of 1.28.
In step S3, common excipients including a sweetener and a flavoring agent were added to the above thick paste, mixed uniformly, a resulting mixture was granulated, dried, and sized to obtain the final product.

### Example 2

This example provides a traditional Chinese medicine composition for treating pediatric atopic dermatitis, which was prepared by the following steps:
S1. The following Chinese medicinal materials were collected as raw materials: 8 g of Atractylodes Rhizoma, 6 g of Lonicerae Japonicae Flos, 6 g of Phellodendri Chinensis Cortex, 8 g of Coicis Semen, 5 g of Achyranthis Bidentatae Radix from Huaiqing region, 6 g of Sophorae Flaves Centis Radix, 6 g of Dictamni Cortex, 6 g of Kochiae Fructus, 6 g of Cicadae Periostracum, 3 g of Glycyrrhizae Radix et Rhizoma; the above raw materials were added to 12 times an amount of water and immersed for 1.5 hrs, then heated under reflux for extraction twice, 2.5 hrs each time, and filtered, and the two filtrates were combined.
In step S2, the combined filtrate was concentrated under a reduced pressure at a temperature of 65°C and a vacuum degree of -0.04 MPa to obtain a clear paste having a relative density of 1.15. The clear paste was cooled, and ethanol was added to and mixed with the clear paste until an ethanol content reached 60%, a resulting mixture was allowed to stand for 36 hrs. A supernatant was collected, filtered, ethanol was recovered from a resulting filtrate, which was then subjected to concentrating under a reduced pressure at a temperature of 70°C and a vacuum degree of -0.06 MPa to yield a thick paste having a relative density of 1.25.
In step S3, common excipients including a sweetener and a flavoring agent were added to the above thick paste, mixed uniformly, a resulting mixture was granulated, dried, and sized to obtain the final product.

### Example 3

This example provides a traditional Chinese medicine composition for treating pediatric atopic dermatitis, which was prepared by the following steps:
In step S1, the following Chinese medicinal materials were collected as raw materials: 8 g of Atractylodes Rhizoma, 8 g of Lonicerae Japonicae Flos, 4 g of Phellodendri Chinensis Cortex, 4 g of Coicis Semen, 5 g of Achyranthis Bidentatae Radix from Huaiqing region, 8 g of Sophorae Flaves Centis Radix, 4 g of Dictamni Cortex, 8 g of Kochiae Fructus, 6 g of Cicadae Periostracum, 4 g of Glycyrrhizae Radix et Rhizoma; the above raw materials were added to 14 times an amount of water and immersed for 1.5 hrs, then heated under reflux for extraction twice, 3 hrs each time, and filtered, and the two filtrates were combined.
In step S2, the combined filtrate was concentrated under a reduced pressure at a temperature of 65°C and a vacuum degree of -0.04 MPa to obtain a clear paste having a relative density of 1.10. The clear paste was cooled, and ethanol was added to and mixed with the clear paste until an ethanol content reached 60%, a resulting mixture was allowed to stand for 36 hrs. A supernatant was collected, filtered, ethanol was recovered from a resulting filtrate, which was then subjected to concentrating under a reduced pressure at a temperature of 75°C and a vacuum degree of -0.06 MPa to yield a thick paste having a relative density of 1.35.
In step S3, common excipients including a bitterness inhibitor and a flavoring agent were added to the above thick paste, mixed uniformly, a resulting mixture was granulated, dried, and sized to obtain the final product.

### Example 4

This example provides a traditional Chinese medicine composition for treating pediatric atopic dermatitis, which was prepared by the following steps:
In step S1, the following Chinese medicinal materials were collected as raw materials: 7 g of Atractylodes Rhizoma, 10 g of Lonicerae Japonicae Flos, 5 g of Phellodendri Chinensis Cortex, 8 g of Coicis Semen, 5 g of Achyranthis Bidentatae Radix from Huaiqing region, 6 g of Sophorae Flaves Centis Radix, 4 g of Dictamni Cortex, 4 g of Kochiae Fructus, 4 g of Cicadae Periostracum, 2 g of Glycyrrhizae Radix et Rhizoma; the above raw materials were added to 9 times an amount of water and immersed for 2 hrs, then heated under reflux for extraction twice, 3 hrs each time, and filtered, and the two filtrates were combined.
In step S2, the combined filtrate was concentrated under a reduced pressure at a temperature of 80°C and a vacuum degree of -0.09 MPa to obtain a clear paste having a relative density of 1.10. The clear paste was cooled, and ethanol was added to and mixed with the clear paste until an ethanol content reached 70%, a resulting mixture was allowed to stand for 12 hrs. A supernatant was collected, filtered, ethanol was recovered from a resulting filtrate, which was then subjected to concentrating under a reduced pressure at a temperature of 75°C and a vacuum degree of -0.08 MPa to yield a thick paste having a relative density of 1.32.
In step S3, common excipients including a bitterness inhibitor and a flavoring agent were added to the above thick paste, mixed uniformly, a resulting mixture was granulated, dried, and sized to obtain the final product.

### Example 5

This example provides a traditional Chinese medicine composition for treating pediatric atopic dermatitis, which was prepared by the following steps:
In step S1, the following Chinese medicinal materials were collected as raw materials: 6 g of Atractylodes Rhizoma, 8 g of Lonicerae Japonicae Flos, 6 g of Phellodendri Chinensis Cortex, 6 g of Coicis Semen, 6 g of Achyranthis Bidentatae Radix from Huaiqing region, 5 g of Sophorae Flaves Centis Radix, 6 g of Dictamni Cortex, 6 g of Kochiae Fructus, 5 g of Cicadae Periostracum, 5 g of Glycyrrhizae Radix et Rhizoma; the above raw materials were added to 10 times an amount of water and immersed for 2 hrs, then heated under reflux for extraction twice, 2 hrs each time, and filtered, and the two filtrates were combined.
In step S2, the combined filtrate was concentrated under a reduced pressure at a temperature of 80°C and a vacuum degree of -0.09 MPa to obtain a clear paste having a relative density of 1.12. The clear paste was cooled, and ethanol was added to and mixed with the clear paste until an ethanol content reached 80%, a resulting mixture was allowed to stand for 12 hrs. A supernatant was collected, filtered, ethanol was recovered from a resulting filtrate, which was then subjected to concentrating under a reduced pressure at a temperature of 75°C and a vacuum degree of -0.08 MPa to yield a clear paste having a relative density of 1.25. The final clear paste was then directly subjected to on-step granulation and dried.

### Example 6

This example provides a traditional Chinese medicine composition for treating pediatric atopic dermatitis, which was prepared by the following steps:
In step S1, the following Chinese medicinal materials were collected as raw materials: 8 g of Atractylodes Rhizoma, 7 g of Lonicerae Japonicae Flos, 6 g of Phellodendri Chinensis Cortex, 5 g of Coicis Semen, 6 g of Achyranthis Bidentatae Radix from Huaiqing region, 5 g of Sophorae Flaves Centis Radix, 5 g of Dictamni Cortex, 6 g of Kochiae Fructus, 6 g of Cicadae Periostracum, 4 g of Glycyrrhizae Radix et Rhizoma; the above raw materials were added to 10 times an amount of water and immersed for 2 hrs, then heated under reflux for extraction twice, 2 hrs each time, and filtered, and the two filtrates were combined.
In step S2, the combined filtrate was concentrated under a reduced pressure at a temperature of 70°C and a vacuum degree of -0.06 MPa to obtain a clear paste having a relative density of 1.10. The clear paste was cooled, and ethanol was added to and mixed with the clear paste until an ethanol content reached 90%, a resulting mixture was allowed to stand for 15 hrs. A supernatant was collected, filtered, ethanol was recovered from a resulting filtrate, which was then subjected to concentrating under a reduced pressure at a temperature of 70°C and a vacuum degree of -0.06 MPa to yield a clear paste having a relative density of 1.20. The final clear paste was then directly subjected to on-step granulation and dried.

### Example 7

This example provides a traditional Chinese medicine composition for treating pediatric atopic dermatitis, which was prepared by the following steps:
In step S1, the following Chinese medicinal materials were collected as raw materials: 8 g of Atractylodes Rhizoma, 6 g of Lonicerae Japonicae Flos, 4 g of Phellodendri Chinensis Cortex, 8 g of Coicis Semen, 6 g of Achyranthis Bidentatae Radix from Huaiqing region, 4 g of Sophorae Flaves Centis Radix, 4 g of Dictamni Cortex, 6 g of Kochiae Fructus, 6 g of Cicadae Periostracum, 5 g of Glycyrrhizae Radix et Rhizoma; the above raw materials were added to 10 times an amount of water and immersed for 3 hrs, then heated under reflux for extraction twice, 1.5 hrs each time, and filtered, and the two filtrates were combined.
In step S2, the combined filtrate was concentrated under a reduced pressure at a temperature of 70°C and a vacuum degree of -0.07 MPa to obtain a clear paste having a relative density of 1.10. The clear paste was cooled, and ethanol was added to and mixed with the clear paste until an ethanol content reached 90%, a resulting mixture was allowed to stand for 20 hrs. A supernatant was collected, filtered, ethanol was recovered from a resulting filtrate, which was then subjected to concentrating under a reduced pressure at a temperature of 70°C and a vacuum degree of -0.07 MPa to yield a clear paste having a relative density of 1.10. The final clear paste was then directly subjected to on-step granulation and dried.

### Experimental Example 1

This experimental example utilizes the traditional Chinese medicine composition prepared in Example 1 to investigate the effect of the above-mentioned traditional Chinese medicine composition on DNCB-induced atopic dermatitis.
1. Animals and grouping: Sixty 6-8 week old BALB/c mice were randomly divided into a normal group, a model group, a positive drug group, and low, medium, and high dose groups of the test substance, with 10 mice in each group.
2. Modeling and drug administration: after grouping, the skin on the backs of all animals was shaved in an area of 2 cm*4 cm. On the first day of modeling, except for the normal group, 150 µL of 1% DNCB was applied to the backs of all other groups, and 20 µL of 1% DNCB was applied to the backs of the right ears of all other groups, for two consecutive days. On days 7, 9, 11, and 13 of modeling, except for the normal group, 100 µL of 0.2% DNCB solution was applied to the backs of all other groups, and 10 µL of 0.2% DNCB solution was applied to the backs of the right ears of all other groups, to stimulate and maintain atopic dermatitis symptoms in mice. In the early stage, edema, erythema, and crusting accompanied by scratching appeared on the backs of the mice. In the later stage, dark red spots and scaling appeared on the backs of the mice, which was considered a successful modeling. Starting on day 15 of the experiment, the normal group and the model group were given the solvent, the positive control group was given prednisolone 10 mg/kg by gavage, and the test substance group was given different doses of the test substance (low, medium, and high doses were 10 g crude drug/kg, 20 g crude drug/kg and 40 g crude drug/kg, respectively) for 14 consecutive days. After the administration was completed, the patients were euthanized and the samples were collected.

### 3. Detection Indicators:

(1) Skin Lesion Score: On day 14 after drug administration, the skin on the back of mice was observed visually, and the severity of skin lesions was scored according to the Scoring of Clinical Atopic Dermatitis (SCORAD) criteria. Standards: Severe erythema, edema, scaling, and epidermal shedding: 3 points; Moderate erythema, edema, scaling, and epidermal shedding: 2 points; Mild erythema, edema, scaling, and epidermal shedding: 1 point; Normal skin: 0 points.
(2) Scratching frequency detection: the number of scratches in mice on day 14 after drug administration was recorded. Mice were placed alone in a quiet environment, and a camera recorded the number of times each mouse scratched its ears, back, and abdomen within 30 minutes. A single scratch was defined as the mouse lifting its paw and continuously scratching its ears and abdomen until it lowered its paw or licked its abdomen.
(3) Right ear thickness detection: after drug administration, the thickness of the right ear of each mouse was measured using calipers.
(4) Spleen and thymus index detection: after euthanasia, the spleen and thymus were weighed. Spleen index = spleen weight (mg) / body weight (g) * 10, thymus index = thymus weight (mg) / body weight (g) * 10.
(5) Skin pathology examination: A suitable amount of skin from the back lesions of mice was taken, fixed in 4% paraformaldehyde solution for 24 hrs, then dehydrated in ethanol solution for 5 minutes using a gradient method, cleared with xylene, embedded in paraffin, and cut into 4 µm thin sections. Hematoxylin-eosin staining was performed, and the sections were mounted with resin. The pathological morphological changes of the sections were observed under a light microscope to observe the degree of epidermal hyperplasia, inflammation, and mast cell infiltration.

### 4. Results:

(1) Compared with the normal group, the model group showed obvious erythema, edema, scaling, or epidermal shedding. Compared with the model group, the skin lesions in the positive drug group and test substance group of different doses were improved, with skin crusting, no erythema, no bleeding, normal hair growth, and a decrease in skin lesion score.
(2) Compared with the normal group, the model group exhibited significant scratching behavior. Compared with the model group, the number of scratches was significantly reduced in the positive drug group and the test substance group of different doses.
(3) Compared with the normal group, the thickness of the right ear in the model group was significantly increased. Compared with the model group, the thickness of the right ear in the positive drug group and the test substance group of different doses was significantly reduced, and edema was decreased.
(4) Compared with the normal group, the thymus index and spleen index of the model group were significantly increased. Compared with the model group, the thymus index and spleen index of the positive drug group and the test substance group of different doses were decreased. The increase in spleen index may be related to the over-activated B lymphocytes.
(5) The skin tissue of mice in the normal group was of normal thickness and clear layers. The hair follicles and sebaceous glands in the dermis were intact, and no obvious edema or inflammatory cell infiltration was observed. The epidermis of mice in the model group was significantly thickened, the sebaceous glands were reduced, no neatly arranged hair follicles were observed, epidermal cells were necrotic, sloughed off, ulcerated, and defective, the intercellular spaces and layers were unclear, the epidermal cells were incompletely/excessively keratinized, the density was poor, condensed cell nuclei were visible in the stratum corneum, and inflammatory cell infiltration was visible in the dermis, with epidermal ridges extending downwards. After drug intervention, the epidermal structure of mice was intact but the thickness increased. A few eosinophils and lymphocytes were visible in the dermis. The incomplete keratinization of the skin was improved, residual cell nuclei were visible in the thickened stratum corneum, the granular layer was not obvious, and the skin showed significant improvement.

5. Conclusion: The traditional Chinese medicine composition of the present application can significantly reduce the number of scratches in mice with atopic dermatitis, inhibit skin itching, and alleviate erythema, edema, and skin inflammation caused by atopic dermatitis, thus having a therapeutic effect on atopic dermatitis in mice.

### Experimental Example 2

The traditional Chinese medicine composition prepared in Example 1 of the present application were applied clinically.

### 1. Research Subjects

The traditional Chinese medicine composition was applied to 60 clinical observation cases, including 28 males and 32 females, who were randomly divided into a treatment group (n=30) and a control group (n=30). The patients ranged in age from 2 to 18 years and had a disease duration of 2 to 14 years. Among them, 12 patients had a clear history of hereditary allergies.

### 2. Diagnostic basis

### Western Medicine Diagnostic Criteria

Referring to the William Schaffner diagnostic criteria for Alzheimer's disease, the main characteristics are as follows: a history of pruritus, plus three or more of the following criteria: (1) History of involvement of flexural skin, including the cubital fossa, popliteal fossa, anterior ankle, or around the neck; (2) Visible dermatitis on flexural surfaces; (3) Onset before age 2; (4) History of asthma or allergic rhinitis; (5) History of generalized dry skin.

### Traditional Chinese Medicine Diagnostic Criteria

Referring to the diagnostic criteria for eczema in the "Standards for Diagnosis and Efficacy of Diseases and Syndromes in Traditional Chinese Medicine" issued by the State Administration of Traditional Chinese Medicine: Eczema is a skin condition caused by constitutional hypersensitivity, which allows wind, dampness, and heat pathogens to lodge in the skin. It is clinically characterized by symmetrical distribution of polymorphic rashes, exudation, intense itching, and recurrent episodes.

### 3. Usage and Dosage

Treatment group: Patients took the medicine prepared from the traditional Chinese medicine composition of the present application by dissolving it in warm water. Patients weighing over 50 kg were given the prescribed dosage; patients weighing 30-50 kg were given 2/3 of the prescribed dosage; and patients weighing less than 30 kg were given 1/3 of the prescribed dosage.

Control group: Patients took loratadine syrup (Wante Pharmaceutical (Hainan) Co., Ltd., batch number H20070001) orally. Patients weighing ≤20 kg took 5 ml daily, and patients weighing >20 kg took 10 ml daily.

### 4. Efficacy standards

According to the diagnostic and therapeutic standards for eczema in the "Standards for Diagnosis and Treatment of Diseases in Traditional Chinese Medicine" issued by the State Administration of Traditional Chinese Medicine:
(1) Basically cured: All skin lesions have subsided, and symptoms such as itching have disappeared, with an efficacy index of 90%-100%;
(2) Significantly effective: Most skin lesions have subsided, and itching has been significantly reduced, with an efficacy index of 70%-90%;
(3) Effective: Some skin lesions have subsided, and itching symptoms have improved, with an efficacy index of 30%-70%;
(4) Ineffective: Skin lesions have not subsided significantly, and itching has not been reduced or clinical symptoms have worsened, with an efficacy index of <30%.

### 5. The efficacies are shown in Table 1.

**Table 1**

| Group | Cured | Significantly Effective | Effective | Ineffective | Total Effective Rate |
|---|---|---|---|---|---|
| Treatment Group | 8 | 18 | 3 | 1 | 86% |
| Control Group | 7 | 14 | 6 | 3 | 70% |

| | | | | | |
|---|---|---|---|---|---|
| Note: Overall effective rate = (Nearly cured cases + Significantly effective cases) / Total number of cases × 100% | | | | | |

### 6. Typical Cases

Case Example 1: Xu, male, 12 years old. Chief complaint: Scattered maculopapular rash all over the body for more than half a month. More than half a month ago, the child developed a generalized red maculopapular rash without any obvious cause, accompanied by itching. After taking the medicine prepared from the traditional Chinese medicine composition of the present application for four courses of treatment, the color of the skin lesions lightened, occasional itching occurred, and no new rashes appeared. After continuing to take the medicine for two more courses of treatment, all skin lesions disappeared, and the itching and other symptoms disappeared, resulting in a cure.

Case Example 2: Ye, male, 4 years old. Chief complaint: Maculopapular rashes all over the body for more than half a year. Upon examination, red maculopapular rashes were found on the face, gradually developing into vesicles, accompanied by itching. After rupture, erosion, exudation, and crusting occurred. Red maculopapular rashes were scattered on the trunk and limbs, accompanied by significant itching. Diagnosis: Atopic dermatitis. After taking the medicine prepared from the traditional Chinese medicine composition of the present application for 8 courses of treatment, the erythema faded, the exudation of the skin lesions significantly decreased, and the itching lessened. After continuing to take the medicine for 6 more courses of treatment, the macules disappeared, the rashes subsided, the crusts fell off, and the patient recovered completely.

The embodiments described in the above are part of, rather than all of, the embodiments of the present application. The detailed description of the embodiments of the present application is not intended to limit the scope of the claims, but merely to illustrate optional embodiments of the present application. All other embodiments obtained by those skilled in the art without making inventive efforts based on the embodiments in the present application fall within the scope of protection of the present application.

## Claims

1. A traditional Chinese medicine composition for treating pediatric atopic dermatitis, **characterized by** comprising the following components of traditional Chinese medicinal raw materials by mass: 4 to 8 parts of an Atractylodes Rhizoma, 6 to 10 parts of a Lonicerae Japonicae Flos, 4 to 8 parts of a Phellodendri Chinensis Cortex, 4 to 8 parts of a Coicis Semen, 4 to 8 parts of an Achyranthis Bidentatae Radix, 2 to 8 parts of a Sophorae Flaves Centis Radix, 4 to 8 parts of a Dictamni Cortex, 4 to 8 parts of a Kochiae Fructus, 2 to 8 parts of a Cicadae Periostracum, and 2 to 8 parts of a Glycyrrhizae Radix et Rhizoma.

2. The traditional Chinese medicine composition for treating pediatric atopic dermatitis according to claim 1, wherein the traditional Chinese medicine composition comprises the following components of traditional Chinese medicinal raw materials by mass: 6 parts of the Atractylodes Rhizoma, 8 parts of the Lonicerae Japonicae Flos, 6 parts of the Phellodendri Chinensis Cortex, 6 parts of the Coicis Semen, 6 parts of the Achyranthis Bidentatae Radix, 5 parts of the Sophorae Flaves Centis Radix, 6 parts of the Dictamni Cortex, 6 parts of the Kochiae Fructus, 5 parts of the Cicadae Periostracum, and 5 parts of the Glycyrrhizae Radix et Rhizoma.

3. The traditional Chinese medicine composition for treating pediatric atopic dermatitis according to claim 1 or 2, wherein the Achyranthis Bidentatae Radix is Achyranthis Bidentatae Radix from Huaiqing region.

4. A method for preparing the traditional Chinese medicine composition for treating pediatric atopic dermatitis according to any one of claims 1-3, **characterized by** comprising the following steps:
step S1, immersing the traditional Chinese medicinal raw materials in water, heating under reflux for extraction, and filtering a resulting mixture to collect a filtrate; and
step S2, concentrating the filtrate into a clear paste, cooling the clear paste, adding ethanol, letting a resulting mixture stand, collecting a supernatant, recovering ethanol, and performing a second concentrating on a resulting liquid to obtain a traditional Chinese medicine paste; and granulating the traditional Chinese medicine paste to obtain a product of the traditional Chinese medicine composition for treating pediatric atopic dermatitis.

5. The method for preparing the traditional Chinese medicine composition for treating pediatric atopic dermatitis according to claim 4, wherein
in step S1, a ratio of the traditional Chinese medicinal raw materials to water is 1:(6-14), an immersing temperature is room temperature, and the immersing time is 0.5 to 3 hrs; and
the heating under reflux for extraction is performed twice, for 1 to 3 hrs each time, and filtrates obtained from the twice heating under reflux for extraction are combined and subjected to concentrating in step S2.

6. The method for preparing the traditional Chinese medicine composition for treating pediatric atopic dermatitis according to claim 5, wherein in step S2, the concentrating is performed under a reduced pressure, a vacuum degree of the concentrating is -0.09 MPa to - 0.04 MPa, a temperature the concentrating is 60 to 80°C, and a clear paste obtained from the concentrating has a relative density of 1.10 to 1.25.

7. The method for preparing the traditional Chinese medicine composition for treating pediatric atopic dermatitis according to claim 4, wherein the resulting mixture after adding ethanol in step S2 has an ethanol content of 30-80%, and a standing time is 8 hrs to 48 hrs.

8. The method for preparing the traditional Chinese medicine composition for treating pediatric atopic dermatitis according to claim 6, wherein the second concentrating in step S2 is performed under a reduced pressure, the traditional Chinese medicine paste obtained from the second concentrating is a thick paste having a relative density of 1.25 to 1.36, an excipient is added to and mixed with the thick paste, granulated, followed by drying to form the product of the traditional Chinese medicine composition.

9. The method for preparing the traditional Chinese medicine composition for treating pediatric atopic dermatitis according to claim 8, wherein the excipient comprises: a sweetener, a flavoring agent, and/or a bitterness inhibitor.

10. The method for preparing the traditional Chinese medicine composition for treating pediatric atopic dermatitis according to claim 6, wherein
the second concentrating in step S2 is performed under a reduced pressure, the traditional Chinese medicine paste obtained from the second concentrating is a clear paste having a relative density of 1.05 to 1.25, which is granulated and dried to obtain a product of the traditional Chinese medicine composition product.
